# EUROPÄISCHE PATENTANMELDUNG

(11) **EP 2 452 612 A1**
(43) Veröffentlichungstag der Anmeldung: **16.05.2012**
(21) Anmeldenummer: 11188396.3
(22) Anmeldetag: 09.11.2011
(51) Int. Cl.: A61B 1/06, A61B 1/24, A61B 1/247, A61B 5/00, A61B 1/00

(54) **Zahnärztliches Gerät mit handgehaltenem Instrument und Lichtquelle**

(30) Priorität: 11.11.2010 DE 102010043795
(71) Anmelder: Kaltenbach & Voigt GmbH, 88400 Biberach (DE)
(72) Erfinder: Hackel, André, 88400 Biberach (DE); Erdmann, Sven, 89081 Ulm (DE)
(74) Vertreter: Thun, Clemens

(57) **Zusammenfassung**

Bei einem zahnärztlichen Gerät (1) mit einem handgehaltenen Instrument (2), an dessen vorderen Ende ein abnehmbarer Aufsatz (10) mit zumindest einer Lichtquelle (20) angeordnet ist, ist die Lichtquelle (20) innerhalb eines abgeschlossenen Gehäuses (21) in dem Aufsatz (10) angeordnet. Ferner sind zwischen dem Aufsatz (10) und dem Instrument (2) wirksame Stromübertragungsmittel für eine Stromversorgung der Lichtquelle (20) vorgesehen.

## Beschreibung

Die vorliegende Erfindung betrifft ein zahnärztliches Gerät mit einem handgehaltenen Instrument, welches eine Lichtquelle aufweist, die zu Beobachtungs- und/oder Untersuchungszwecken genutzt wird. Insbesondere betrifft die vorliegende Erfindung eine sogenannte Intraoralkamera oder ein System zum Transilluminieren von Zähnen.

In der Medizin, insbesondere der Zahnmedizin werden vermehrt Diagnosesysteme eingesetzt, welche auf optischen Prinzipien beruhen. Grund hierfür ist, dass derartige Geräte üblicherweise berührungslos, also insbesondere schmerzfrei die Erstellung einer Diagnose ermöglichen und darüber hinaus auch oftmals optische Bilder zur Verfügung stellen, anhand welcher eventuell erforderliche therapeutische Maßnahmen dem Patienten anschaulich und damit besser vermittelt werden können. Beispielsweise kommen in der Zahnmedizin sogenannte Intraoralkameras zum Einsatz, welche ein Handstück beinhalten, dessen vorderer Endbereich in den Mund eines Patienten eingeführt wird. In diesem Endbereich befindet sich in der Regel ein Lichteintritts- bzw. Sichtfenster für die Kameraoptik, von dem ausgehend das Bild des zu untersuchenden Objekts einer Erfassungseinrichtung, beispielsweise einem CCD-Chip übermittelt wird.

Eine derartige Intraoralkamera kann ferner zu einem System zum Transilluminieren von Zähnen erweitert werden, wie es u. a. aus der DE 10 2006 041 020 A1 der Anmelderin bekannt ist. Hierbei wird der zu untersuchende Zahn mit Licht innerhalb eines bestimmten Wellenlängenbereichs bestrahlt, wobei dann ein optisches Bild des durch die Untersuchungsstrahlung illuminierten Zahns erfasst und bewertet wird. Da kariöse Stellen im Zahn das Licht anders streuen als gesundes Zahngewebe, können derartige Stellen bei Beobachtung des Zahns mit Hilfe einer Kamera identifiziert werden, wobei bei entsprechender Ausgestaltung des Systems sogar eine zuverlässigere Kariesdiagnose möglich ist als dies bei einer klassischen Röntgenuntersuchung der Fall ist.

Sowohl im Fall der klassischen Intraoralkamera als auch im Fall des Systems zum Transilluminieren von Zähnen ist für den Erhalt guter und aussagekräftiger Aufnahmen unerlässlich, Licht in effektiver Weise auf den zu beobachtenden bzw. zu untersuchenden Zahn zu richten. Gleichzeitig sollte allerdings der vordere Endbereich der Kamera bzw. des handgehaltenen Instruments kompakt gestaltet sein, da sich aufgrund der beengten Verhältnisse innerhalb des Mundraums des Patienten ohnehin Schwierigkeiten bei der Handhabung ergeben. Aus diesem Grund ist üblicherweise eine Anordnung der Lichtquelle bzw. Lichtquellen unmittelbar im vorderen Endbereich des Instruments nicht möglich. Neben den beengten Platzverhältnissen spricht darüber hinaus auch die von den Lichtquellen generierte Wärme gegen eine Anordnung im vorderen Endbereich des Instruments. Diese ist nicht zu vernachlässigen und könnte von dem Patienten als unangenehm empfunden werden, weshalb es bislang üblich war, die Lichtquellen eher im zentraleren Bereich der Griffhülse des Instruments anzuordnen und dann das Licht mittels Lichtleitern zu einer entsprechenden Lichtaustrittsstelle zu lenken. Diese Ausgestaltung ist auch insofern von Vorteil, als hierbei die Lichtquellen geschützt vor äußeren Einflüssen innerhalb des Instruments angeordnet werden können. Dies ist deshalb wichtig, da derartige Untersuchungsinstrumente aus hygienischen Gründen regelmäßig gereinigt und sterilisiert werden müssen, wobei sie hierzu teilweise verhältnismäßig hohen Temperaturen ausgesetzt werden, welche zu einer Beschädigung der Lichtquellen führen könnten.

Da auf der anderen Seite die Übertragung von Licht mittels Lichtleitern immer auch mit einem gewissen Verlust von Licht verbunden ist, wäre es wünschenswert, die Lichtquellen möglichst nahe an dem Lichtaustrittsbereich des Instruments anzuordnen. Der vorliegenden Erfindung liegt die Aufgabe zugrunde, eine neuartige Lösung anzugeben, die dies ermöglicht und bei der gleichzeitig die oben beschriebenen Probleme vermieden werden.

Die Aufgabe wird durch ein zahnärztliches Gerät, welches die Merkmale des Anspruchs 1 aufweist, gelöst. Vorteilhafte Weiterbildungen der Erfindung sind Gegenstand der abhängigen Ansprüche.

Die erfindungsgemäße Lösung beruht auf dem Gedanken, die Lichtquelle, bei der es sich beispielsweise um eine LED oder um eine Laserdiode handeln kann, in einen aufsteckbaren Aufsatz des zahnärztlichen handgehaltenen Geräts zu integrieren, wobei insbesondere die Anordnung der Lichtquelle derart ist, dass der Aufsatz mit der Lichtquelle insgesamt sterilisierbar ist.

Hierzu wird gemäß der vorliegenden Erfindung ein zahnärztliches Gerät mit einem handgehaltenen Instrument vorgeschlagen, an dessen vorderen Ende ein abnehmbarer Aufsatz mit zumindest einer Lichtquelle angeordnet ist, wobei die Lichtquelle innerhalb eines abgeschlossenen Gehäuses in dem Aufsatz angeordnet ist und ferner zwischen dem Aufsatz und dem Instrument wirksame Stromübertragungsmittel für eine Stromversorgung der Lichtquelle vorgesehen sind.

Dadurch, dass der Aufsatz abnehmbar ist, besteht die Möglichkeit, diesen getrennt von dem restlichen zahnärztlichen Instrument zu reinigen und/oder zu sterilisieren, sodass insbesondere die besonders sensiblen elektronischen Komponenten des Instruments nicht den Belastungen des Sterilisiervorgangs ausgesetzt sind. Ferner ist dadurch, dass die Lichtquelle innerhalb eines geschlossenen Gehäuses in dem Aufsatz angeordnet ist, sichergestellt, dass die Lichtquelle ebenfalls nicht beschädigt wird. Gleichzeitig ist sie nunmehr allerdings verhältnismäßig nahe an der Lichtaustrittsstelle angeordnet, sodass evtl. Verluste bei der Übertragung mittels Lichtleitern nicht allzu hoch sind. Das von der Lichtquelle abgegebene Licht kann dementsprechend effektiv zu Beobachtungs-und/oder Untersuchungszwecken genutzt werden.

Vorzugsweise ist die Lichtquelle auf einem Sockel angeordnet, der gemeinsam mit einer zugehörigen Abdeckung einen abgedichteten Innenraum zur Aufnahme der Lichtquelle umschließt. Hierbei kann die Abdeckung einen transparenten Bereich zur Abgabe des von der Lichtquelle emittierten Lichts aufweisen, wobei vorzugsweise der transparente Bereich ein optisches Element, insbesondere eine Linse zur Beeinflussung des abgegebenen Lichts bildet.

Die Stromübertragung zwischen dem Instrument und dem Aufsatz kann beispielsweise durch einfache Kontakte gebildet sein. Denkbar wäre allerdings auch eine induktive Stromübertragung, welche es zusätzlich ermöglicht, dass der Aufsatz bewegbar, insbesondere drehbar gegenüber dem Instrument angeordnet ist. Da derartige Instrumente in der Regel über einen Versorgungsschlauch bzw. ein Kabel mit einer Zentraleinheit oder Versorgungseinheit verbunden sind, kann in diesem Fall die Handhabung ggf. vereinfacht werden.

Bei dem erfindungsgemäßen zahnärztlichen Gerät kann es sich beispielsweise um eine Dentalkamera handeln. Es kann allerdings auch als Gerät zum Transilluminieren von Zähnen genutzt werden.

Nachfolgend soll die Erfindung anhand der beiliegenden Zeichnung näher erläutert werden. Es zeigen:
- Figur 1: die seitliche Ansicht eines erfindungsgemäßen zahnärztlichen Geräts, welches zum Transilluminieren von Zähnen genutzt wird;
- Figur 2: eine vergrößerte Ansicht des vorderen Endbereichs des Geräts von Figur 1;
- Figur 3: schematisch eine erste Variante zur Stromübertragung zwischen dem Instrument und dem abnehmbaren Aufsatz und
- Figur 4: schematisch eine zweite Variante zur Stromübertragung zwischen Instrument und Aufsatz.

Bevor das erfindungsgemäße zahnärztliche Gerät detaillierter beschrieben wird, soll zunächst das der Untersuchungsmethode zugrunde liegende Verfahren kurz erläutert werden. Dieses Transilluminations-Verfahren beruht darauf, einen Zahn mit sichtbarem Licht zu durchleuchten bzw. zu transilluminieren. Hierzu wird mit Hilfe einer Lichtquelle eine Untersuchungsstrahlung generiert, welche auf den Zahn gerichtet wird. Die Untersuchungsstrahlung liegt dabei üblicherweise in einem Wellenlängenbereich von etwa 550 µm bis 790 µm, z. B. bei etwa 670 µm. Das Gewebe des Zahns blockt diese Untersuchungsstrahlung nicht vollständig ab, sondern erlaubt stattdessen, dass das Licht durch den Zahn hindurchtritt. Hierbei wird die Strahlung teilweise gestreut, wobei insbesondere kariöse Bereiche für eine charakteristische Beeinflussung des Lichts sorgen. Wird der auf diese Weise transilluminierte Zahn aus verschiedenen Blickrichtungen betrachtet, so können diese kariösen Bereiche erkannt werden, da diese etwas dunkler erscheinen. Insbesondere dann, wenn Aufnahmen, die zu verschiedenen Zeitpunkten erstellt wurden, miteinander verglichen werden, kann Karies auf diese Weise verhältnismäßig effektiv und auch frühzeitig erkannt werden.

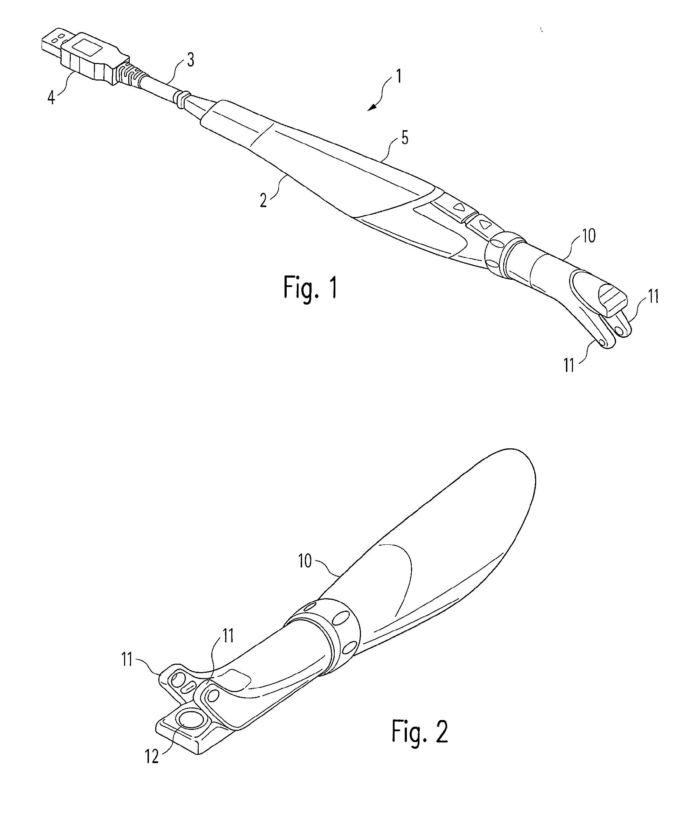

Ein auf diesem Untersuchungsprinzip beruhendes sogenanntes FOTI-(fibre-optik transillumination)-Gerät ist in den Figuren 1 und 2 dargestellt und allgemein mit dem Bezugszeichen 1 versehen. Wesentlicher Bestandteil ist ein handgehaltenes, längliches Instrument 2, das zum Beleuchten des zu untersuchenden Zahns genutzt wird und auch dazu dient, das optische Bild des illuminierten Zahns zu erfassen. Die hierbei gewonnenen Daten werden über ein Kabel 3, an dessen Ende sich ein USB-Stecker 4 befindet, an eine Zentraleinheit, insbesondere einen - nicht dargestellten - PC übermittelt. Hier erfolgt dann eine Darstellung der erstellten Bilder sowie eine Auswertung der Daten, um Karies zu erkennen. Selbstverständlich könnte die Verbindung mit der Zentrale auch anderweitig erfolgen, wobei der USB-Stecker 4 allerdings eine bevorzugte Ausführungsform darstellt. Über diesen erfolgt gleichzeitig auch eine Stromversorgung des Geräts 1.

Das Handinstrument 2 besteht zunächst aus einer länglichen Griffhülse 5, in der die wesentlichen elektronischen Komponenten des Geräts 1 angeordnet sind. Es handelt sich hierbei einerseits um die entsprechende Elektronik zur Kommunikation über den USB-Anschluss mit der Zentrale sowie um Mittel zur Bilderfassung und Aufzeichnung. Hierbei kann es sich beispielsweise um einen CCD-Chip handeln, mit dessen Hilfe ein optisches Bild des Zahns aufgezeichnet wird.

Die optische Bildübertragung zu dem in der Griffhülse 5 befindlichen CCD-Chip erfolgt mit Hilfe eines Aufsatzes 10, der am vorderen Ende der Griffhülse 5 lösbar an dieser angeordnet ist. Dieser Aufsatz 10 dient einerseits zum Beleuchten des zu untersuchenden Zahns 100, wie dies in Figur 2 dargestellt ist. Zum anderen wird mit Hilfe des Aufsatzes 10 ein Bild des illuminierten Zahns 100 erfasst und an die Bilderfassungsmittel, also beispielweise den CCD-Chip auf optischem Wege weitergeleitet. Der Aufsatz 10 weist hierzu an seinem vorderen Ende ein zur Unterseite hin gerichtetes Fenster auf, welches dann über weitere optische Elemente mit den Bilderfassungsmitteln gekoppelt ist. Zur Bildweiterleitung werden in erster Linie Spiegel, Prismen, Linsen und dergleichen verwendet.

Auch die Beleuchtung des Zahns 100 erfolgt - wie bereits erwähnt - über den Aufsatz 10. Hierfür weist dieser an seinem vorderen Ende seitliche Arme 11 auf, welche entsprechend der Darstellung in Figur 2 zur Untersuchung des Zahns 100 derart angeordnet werden, dass sie an den Seitenflächen des Zahns 100 oder dem Zahnfleisch, der sogenannten Gingiva, anliegen. Mit Hilfe des dargestellten Aufsatzes 10 wird also seitlich Licht in den Zahn 100 eingekoppelt, wobei dieser dann von seiner Oberseite her beobachtet wird. Denkbar wäre es allerdings auch, den Aufsatz derart zu gestalten, dass Licht von einer Seite her in den Zahn eingekoppelt wird und der Zahn von der gegenüberliegenden Seite beobachtet wird.

Aus Platzgründen ist eine Anordnung der Lichtquelle zur Erzeugung der Untersuchungsstrahlung unmittelbar in den Seitenarmen 11 nicht möglich. Stattdessen befindet sich die Lichtquelle innerhalb des Aufsatzes 10 und strahlt ihr Licht in entsprechende Lichtleiter ein, mit deren Hilfe dann das Licht zu den Armen 11 weitergeleitet und dort auf den Zahn 100 gerichtet wird. Dadurch, dass die Lichtquelle in dem Aufsatz 10 angeordnet ist, können die entsprechenden Lichtleiter verhältnismäßig kurz gehalten werden, sodass auch die hieraus resultierenden Verluste möglichst gering gehalten werden.

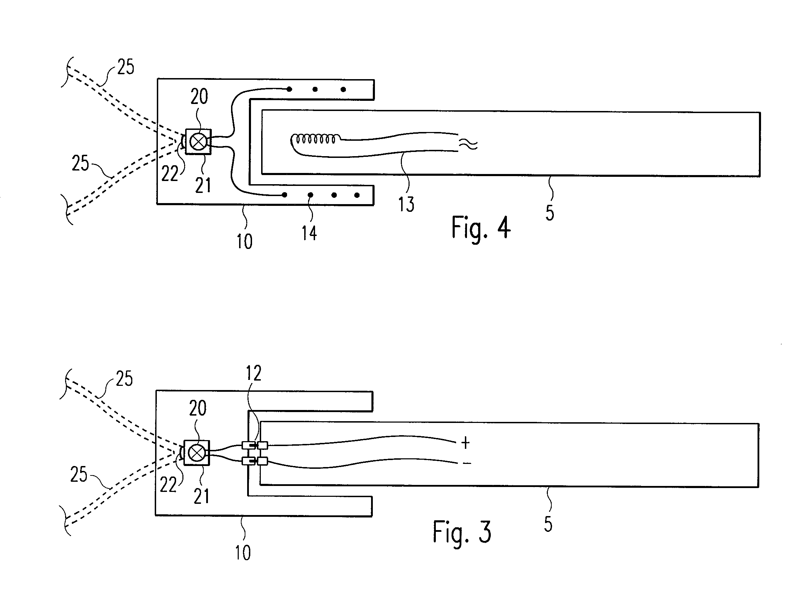

Da aus hygienischen Gründen eine regelmäßige Reinigung und insbesondere auch Desinfektion des vorderen Bereichs des Geräts 1, der in den Mund des Patienten eingeführt wird, unerlässlich ist, ist der Aufsatz 10 lösbar an der Griffhülse 5 des Handinstruments 2 angeordnet. Dies wiederum bedeutet allerdings, dass spezielle Maßnahmen getroffen werden müssen, um einerseits eine entsprechende Stromversorgung der Lichtquelle in dem Aufsatz 10 sicherzustellen und andererseits diese ausreichend zu schützen, um eine Beschädigung während des Sterilisationsvorgangs zu verhindern. Hierfür sind nunmehr entsprechende Maßnahmen vorgesehen, die nachfolgend anhand der Figuren 3 und 4 näher erläutert werden sollen. Beide Figuren zeigen dabei schematisch die Anordnung des Aufsatzes 10 auf der Griffhülse 5.

Die erfindungsgemäße Lösung zeichnet sich dadurch aus, dass die Lichtquelle 20, die vorzugsweise durch eine Laserdiode gebildet ist, derart in den Aufsatz 10 integriert ist, dass dieser ohne Weiteres sterilisiert werden kann ohne dass die Gefahr besteht hierbei die Lichtquelle 20 zu beschädigen. Dies wird dadurch erreicht, dass die Lichtquelle 20 in einem hermetisch abgeschlossenen Gehäuse 21 angeordnet ist, welches an seiner Vorderseite einen transparenten Bereich zur Abgabe des von der Lichtquelle 20 emittierten Lichts aufweist. Insbesondere ist dieser transparente Bereich durch ein optisches Element in Form einer Linse 22 gebildet. Diese ist derart im Strahlengang des Lichts angeordnet, dass eine Bündelung bzw. parallele Ausrichtung der Lichtstrahlen erzielt wird, wodurch diese in äußerst effektiver Weise in die angedeuteten Lichtleiter 25 eingekoppelt werden, welche dann verzweigen und zu den vorderen Endbereichen der Arme 11 führen. Die Linse 22 kann dabei Teil eines transparenten Bereichs des Gehäuses 21 sein oder vollständig den transparenten Bereich bilden.

Die hermetische Abschließung des Gehäuses 21 wird beispielsweise dadurch erreicht, dass die Lichtquelle 20 auf einem Sockel angeordnet ist, der gemeinsam mit einer Abdeckung einen abgedichteten Innenraum umschließt, der zur Aufnahme der Lichtquelle 20 genutzt wird.

Die Stromversorgung der Lichtquelle 20 erfolgt über das handgehaltene Instrument 2, wobei entsprechende Stromübertragungsmittel vorgesehen sind, welche ein Abnehmen des Aufsatzes 10 ermöglichen. Bei der Variante gemäß Figur 3 werden hierzu elektrische Kontakte 12 verwendet, welche in einfacher Weise für eine Verbindung zwischen Griffhülse 5 und Aufsatz 10 sorgen. Alternativ hierzu kann allerdings auch - wie in Figur 4 dargestellt - eine induktive Kopplung zwischen dem Gerät 2 und der Griffhülse 5 genutzt werden. Hierzu befindet sich in der Griffhülse 5 eine stromdurchflossene Anregerspule 13 und in dem Aufsatz 10 eine Induktionsspule 14, welche elektrisch mit der Lichtquelle 20 verbunden ist. Diese zweite Variante bringt den Vorteil mit sich, dass der Aufsatz 10 gegenüber der Griffhülse 5 des Handinstruments 2 frei gedreht werden kann, was ggf. Vorteile in der Handhabung des Geräts mit sich bringt.

Letztendlich wird durch die erfindungsgemäße Lösung der Vorteil erzielt, dass das Licht einer Lichtquelle sehr effektiv an die erforderliche Lichtaustrittsstelle befördert werden kann, gleichzeitig allerdings die Möglichkeit besteht, den Aufsatz regelmäßig zu reinigen und zu sterilisieren, ohne dass eine Beschädigung der Lichtquelle zu befürchten wäre.

Darauf hinzuweisen ist ferner, dass die vorliegende Erfindung nicht auf Geräte bzw. Systeme zum Transilluminieren von Zähnen beschränkt ist. Es wäre auch denkbar, eine entsprechende Ausgestaltung bei klassischen Intraoralkameras vorzusehen. Auch hier kommen die oben genannten Vorteile, die sich aus der erfindungsgemäßen Lösung ergeben, zum Tragen.

## Patentansprüche

1. Zahnärztliches Gerät (1) mit einem handgehaltenen Instrument (2), an dessen vorderen Ende ein abnehmbarer Aufsatz (10) mit zumindest einer Lichtquelle (20) angeordnet ist,
wobei die Lichtquelle (20) innerhalb eines abgeschlossenen Gehäuses (21) in dem Aufsatz (10) angeordnet ist und ferner zwischen dem Aufsatz (10) und dem Instrument (2) wirksame Stromübertragungsmittel für eine Stromversorgung der Lichtquelle (20) vorgesehen sind.

2. Zahnärztliches Gerät nach Anspruch 1,
**dadurch gekennzeichnet,**
**dass** die Lichtquelle (20) durch eine LED oder eine Laserdiode gebildet ist.

3. Zahnärztliches Gerät nach Anspruch 1 oder 2,
**dadurch gekennzeichnet,**
**dass** die Lichtquelle (20) auf einem Sockel angeordnet ist, der gemeinsam mit einer Abdeckung einen abgedichteten Innenraum zur Aufnahme der Lichtquelle (20) umschließt.

4. Zahnärztliches Gerät nach Anspruch 3,
**dadurch gekennzeichnet,**
**dass** die Abdeckung einen transparenten Bereich zur Abgabe des von der Lichtquelle (20) emittierten Lichts aufweist.

5. Zahnärztliches Gerät nach Anspruch 4,
**dadurch gekennzeichnet,**
**dass** der transparente Bereich ein optisches Element, insbesondere eine Linse (22) zur Beeinflussung des abgegebenen Lichts bildet.

6. Zahnärztliches Gerät nach einem der vorherigen Ansprüche,
**dadurch gekennzeichnet,**
**dass** die zwischen dem Aufsatz (10) und dem Instrument (2) wirksamen Stromübertragungsmittel durch Kontakte (12) gebildet sind.

7. Zahnärztliches Gerät nach einem der Ansprüche 1 bis 5,
**dadurch gekennzeichnet,**
**dass** die zwischen dem Aufsatz (10) und dem Instrument (2) wirksamen Stromübertragungsmittel eine induktive Stromübertragung ermöglichen.

8. Zahnärztliches Gerät nach einem der vorherigen Ansprüche,
**dadurch gekennzeichnet,**
**dass** es sich bei dem Gerät um eine Dentalkamera handelt.

9. Zahnärztliches Gerät nach einem der Ansprüche 1 bis 7,
**dadurch gekennzeichnet,**
**dass** es sich bei dem Gerät um ein Gerät zum Transilluminieren von Zähnen (100) handelt.
